Europäisches Patentamt

European Patent Office

Office européen des brevets

(11) Publication number: **0 359 549**
**A1**

## (12) EUROPEAN PATENT APPLICATION

(21) Application number: 89309301.3

(51) Int. Cl.⁵: **A 61 B 1/00**

(22) Date of filing: **13.09.89**

(30) Priority: **13.09.88 US 243898**

(43) Date of publication of application:
**21.03.90 Bulletin 90/12**

(84) Designated Contracting States:
**AT BE CH DE ES FR GB GR IT LI LU NL SE**

(71) Applicant: **VANCE PRODUCTS INCORPORATED TRADING AS COOK UROLOGICAL INCORPORATED**
**1100 West Morgan Street P.O. Box 227**
**Spencer Indiana 47460 (US)**

(72) Inventor: **Bosley, Rodney W., Jr.**
**3997 West Furr Court**
**Bloomington Indiana 47401 (US)**

**Thomson, Paul G.**
**Route 1, Box 24**
**Fillmore Indiana 46128 (US)**

**Pingleton, Edward D.**
**Route 1 Box 202 A**
**Fillmore Indiana 46128 (US)**

(74) Representative: **Hartley, David et al**
**Withers & Rogers 4 Dyer's Buildings Holborn**
**London, EC1N 2JT (GB)**

(54) **Flexible cable construction.**

(57) A flexible cable construction for use in endoscopic/surgical accessory devices or instruments, comprising a continuous coil (12) surrounded by a cylindrical woven braid (14) that has been drawn down snuggly upon and along the entire length of the coil by longitudinal tension and then secured to the coil while under tension, at least at its termini (16, 18).

EP 0 359 549 A1

Description

# FLEXIBLE CABLE CONSTRUCTION

## Background of the Invention

This invention relates to and is particularly concerned with a unique flexible cable construction for use in medical endoscopic surgical accessory devices or instruments.

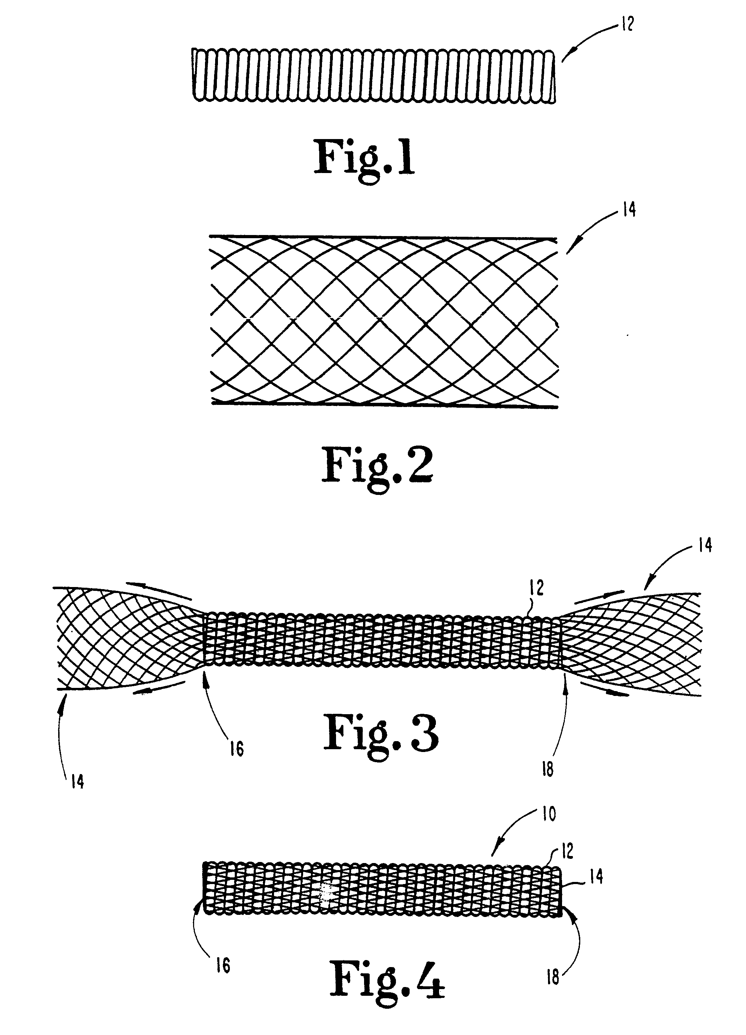

The use of wire guides within the flexible shafts of endoscopic/surgical accessories or instruments is well known. One typical and preferred construction for such wire guides has been a continuous stainless steel wire coil 12 (FIG. 1), which has an open cylindrical lumen (not shown).

Stainless steel wire coils have sufficient flexibility for use as wire guides within the shafts of various endoscopic/surgical accessories or instruments. However, even though constructed from rigid stainless steel wire, such coils lack adequate stability to resist undesirable expansions and compressions. As a consequence, safety wires have been inserted along the full length of the wire guide's lumen, and have then been secured to both ends of the wire guide by soldering, or the like. When in place, the safety wire significantly limits any expansion or compression of the wire guide when in use. Elongation, in fact, is limited to nearly zero, relative to the propensity of the stainless steel wire coil to elongate in use without the presence of a safety wire.

Safety wires have been constructed with various cross-sectional shapes, such as round, flat, or triangular, with a flat-wire design being preferred in the art.

Placing a safety wire within the lumen of continuous wire guides to limit elongation and compression has an undesirable disadvantage. The safety wire takes up so much lumenal space that it greatly limits the ability of the wire guide lumen to accept other wires or other assemblies through the compromised lumen. On the other hand, securing the safety wire to the exterior of the coil wire guide, either at each end only, or by tack soldering the safety wire to the wire guide at increments along the entire length of the wire guide, produces a wire guide with insufficient flexibility to function as desired in endoscopes/surgical accessory devices or instruments.

The invention herein disclosed overcomes the disadvantage of utilizing either interior or exterior attached safety wires to limit the compression and elongation of continuous coil wire guides by providing a novel exterior constraint that substantially limits the compression and elongation of continuous coil wire guides while preserving the flexibility in the combination that is required.

## Summary of the Invention

One embodiment of the present invention is a flexible cable construction for use in endoscopic/surgical accessory devices or instruments, comprising a continuous coil surrounded by a cylindrically woven braid that has been drawn down snuggly upon and along the entire length of the coil by longitudinal tension and permanently secured to the coil at least at its termini.

Another embodiment of the present invention is a method of producing a flexible cable construction for use in endoscopic/surgical accessory devices or instruments, which comprises providing a continuous coil, placing the coil within a cylindrically woven braid, applying longitudinal tension to the woven braid to draw it down snuggly upon and along the entire length of the coil, and then securing the braid to the coil at least at the coil's termini.

It is an object of the present invention to provide a novel flexible cable construction for wire guides used in endoscopic/surgical accessory devices or instruments.

It is a further object of the invention to provide a novel exterior constraint for a coil wire guide to substantially limit the compression and elongation of the wire guide while maintaining the flexibility required.

Related objects and advantages of Applicants' invention will be evident from the following descriptions.

## Brief Description of the Drawings

FIG. 1 is a side elevational view of a continuous stainless steel wire coil 12, having an open cylindrical lumen.

FIG. 2 is a side elevational view of a cylindrically woven stainless steel wire braid 14, having an open cylindrical lumen.

FIG. 3 is a side elevational view of the coil 12 of FIG. 1 within the wire braid 14 of FIG. 2, which has been drawn down snuggly along the entire length of the coil 12 by longitudinal tension upon the braid 14 in the general direction of the arrows.

FIG. 4 is a side elevational view of a preferred embodiment of the flexible cable construction 10 of the present invention.

## Description of the Preferred Embodiment

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

Referring now to the drawings, there is shown in FIG. 4 a preferred embodiment of the flexible cable construction 10 of the present invention. This preferred embodiment is composed of a very flexible, continuous, cylindrical, stainless steel wire coil 12 (FIG. 1), having an outside diameter of about 0.026 inches and an open cylindrical lumen (not shown) of a smaller inside diameter, which coil 12 is typically used as a wire guide for endoscopic/surgical accessory devices or instruments. Surrounding the exterior of coil 12 is a commerically available cylindrical woven stainless steel wire braid 14 (FIG. 2), having an initial outside diameter of about 0.085 inches and an inside diameter of about 0.074 inches. Wire braid 14 also has an open cylindrical lumen (not shown). In this preferred embodiment, the wire braid 14 is slipped over stainless steel coil 12 (FIG. 3), and is then drawn down uniformly and snuggly along the entire length of coil 12 by applying longitudinal tension upon the braid beyond the termini 16 and 18 of coil 12 and in the general direction of the arrows in FIG. 3, until wire braid 14 is fitted snuggly around the coil 12 from one end 16 to the other 18.

The longitudinally tensioned braid 14 (FIG. 3) is then secured to coil 12 at the coil's termini 16 and 18 by conventionally soldering techniques. Alternatively, braid 14 could be secured to coil 12 by welding, or by an adhesive, although soldering and welding are preferred when metal coils and braids are used. Any excess braid 14 beyond the coil's termini 16 and 18 is then cut away.

In this preferred embodiment, the outside diameter of the coil 12 with snuggly fit braid 14 (FIG. 4) is about 0.035 inches.

When drawn down and secured to the coil 12 as described, braid 14 functions somewhat analogous to a "Chinese handcuff" or corset/girdle as it uniformly encases the inner continuous coil 12 and uniformly distributes any and all internally and externally applied stresses upon the coil 12 in an omni-directional manner.

Quite unexpectedly, Applicants discovered that the resulting flexible cable construction 10 (FIG. 4) demonstrates the following significant and substantial attributes over wire coils 12 equipped with safety wires; (1) zero to and insignificant amount of elongation of the inner coil wire 12 or flexible cable construction 10 under normal tensile stress loads; (2) zero to an insignificant amount of compression of the inner coil wire 12 or flexible cable construction 10 under normal compression stress loads; (3) an insignificant compromise in flexibility over that of the wire coil 12 alone along the full length of the flexible cable construction 10, and less of a compromise in flexibility than that experienced with the use of safety wires secured within the wire guide lumen; (4) a uniform strengthening evidenced by an absence of any non-uniform segments that tend to kink or otherwise perform unpredictably, or non-uniformly; and (5) uniformly omni-directional in that the cable construction 10 does not "kick" to one side or the other, but bends uniformly in all directions with constant flexibility and response.

Stainless steel has been the preferred material in testing done to date for both the coil 12 and the woven braid 14, forming a totally stainless steel flexible cable construction 10. However, it is to be understood that all engineering polymers (e.g., plastics, polycarbonates, polyethylenes, polysufones and the like); ceramic materials (e.g., quartz, soda glass, fiberglass composites and the like)) and other metals or metal alloys (e.g., aluminum, mild steel, brass and the like) and composite alloys of metals, polymers and ceramics capable of being coiled and braided would be suitable materials to utilize in constructing the flexible cable construction 10 of the disclosed invention.

In testing done to date, the functional lengths of flexible cable construction 10 of the present invention have ranged from 5 centimeters to 250 centimeters, with a preferred range of lengths between 30 centimeters and 125 centimeters. Also in testing done to date, the functional outside diameters of the flexible cable construction 10 of the present invention has ranged between 0.006 inches up to 0.395 inches, with a preferred range of outside diameters being between 0.026 inches to 0.066 inches. Within these functional limits the dimensions of the wire coil 12 and braid 14 may be varied provided the braid 14 may be drawn down in the manner disclosed over the outside of the coil.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

## Claims

1. A flexible cable construction for use in endoscopic/surgical accessory devices or instruments, comprising a continuous coil surrounded by a cylindrically woven braid that has been drawn down snuggly upon and along the entire length of said coil by longitudinal tension and then permanently secured to said coil at least at its termini.

2. The flexible cable construction of claim 1 wherein said coil is a stainless steel wire coil.

3. The flexible cable construction of claim 2 wherein said coil is a stainless steel continuous coil wire guide.

4. The flexible cable construction of claim 1 wherein said woven braid is a cylindrically woven stainless steel wire braid.

5. The flexible cable construction of claim 1 wherein the outside diameter of said flexible cable construction is about 0.006 inches to 0.395 inches.

6. The flexible cable construction of claim 1 wherein the length of said cable is about 5 to 250 centimeters.

7. A method of producing a flexible cable construction for use in endoscopic/surgical accessory devices or instruments, which comprises providing a continuous coil, placing said coil within a cylindrically woven braid, applying longitudinal tension to said woven braid to draw it down snuggly upon and along the entire length of said coil, and then securing said braid to said coil at least at the coil's termini.

8. The method of claim 1 wherein said coil is a stainless steel wire coil.

9. The method of claim 2 wherein said coil is a stainless steel continuous coil wire guide.

10. The method of claim 1 wherein said woven braid is a cylindrically woven stainless steel wire braid.

12

Fig.1

14

Fig.2

14
12
14

16
18

Fig.3

14

10
12
14

16
18

Fig.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 098 100  (OLYMPUS OPTICAL CO., LTD)<br>* Abstract; page 2, line 25 - page 3, line 3; page 3, line 32 - page 4, line 27; figure 2 * | 1,4,7, 10 | A 61 B   1/00 |
| A | US-A-3 467 101  (T.J. FOGARTY et al.)<br>* Column 2, lines 40-43,57-70; figures 1-3 * | 1-3,7-9 | |
| A | US-A-3 670 721  (M. FUKAMI et al.)<br>* Abstract; column 2, lines 46-60; figure 4 * | 1,4,7, 10 | |
| A | US-A-3 855 897  (N. TAKAHASHI et al.)<br>* Abstract; column 2, lines 19-28,44-67; column 3, lines 5-12; figures 1-7 * | 1,7 | |
| A | DE-A-2 850 021  (OLYMPUS OPTICAL CO., LTD)<br>* Page 7, line 5 - page 8, line 10; figure 2 * | 1,10 | TECHNICAL FIELDS SEARCHED (Int. Cl.5)<br><br>A 61 B |

The present search report has been drawn up for all claims

| Place of search | Date of completion of the search | Examiner |
|---|---|---|
| THE HAGUE | 15-11-1989 | RIEB K.D. |